Europäisches Patentamt

European Patent Office

Office européen des brevets

(11) Numéro de publication : **0 350 398 B1**

(12) **FASCICULE DE BREVET EUROPEEN**

(45) Date de publication du fascicule du brevet :
23.06.93 Bulletin 93/25

(51) Int. Cl.⁵ : **A61K 39/205, C12N 7/00**

(21) Numéro de dépôt : **89401925.6**

(22) Date de dépôt : **05.07.89**

(54) **Vaccin antirabique avirulent.**

(30) Priorité : **05.07.88 FR 8809099**

(43) Date de publication de la demande :
**10.01.90 Bulletin 90/02**

(45) Mention de la délivrance du brevet :
**23.06.93 Bulletin 93/25**

(84) Etats contractants désignés :
**AT BE CH DE ES GR IT LI LU NL SE**

(56) Documents cités :
**EP-A- 0 202 142**
**CHEMICAL ABSTRACTS, vol. 102, no. 17, 29**
**avril 1985, Columbus, OH (US); I.SEIF et al., p.**
**133, no. 143960y**
**CHEMICAL ABSTRACTS, vol. 105, no. 5, 4 août**
**1986, Columbus, OH (US); A.DIALLO, p. 545,**
**no. 40596s**

(72) Inventeur : **Flamand, Anne**
**5 Clos de Monthyon**
**F-9110 Gif-sur-Yvette (FR)**
Inventeur : **Coulon, Patrice**
**17, Allée de la Butte de Rheims**
**F-91120 Palaiseau (FR)**
Inventeur : **Leblois, Hélène**
**Les Chateliers**
**F-35230 Saint Erblon (FR)**
Inventeur : **Lafay, Florence**
**9, Boulevard de Lesseps**
**F-78000 Versailles (FR)**
Inventeur : **Tuffereau, Marie-Christine**
**35, Avenue Jean Moulin**
**F-75014 Paris (FR)**

(74) Mandataire : **Gillard, Marie-Louise et al**
**Cabinet Beau de Loménie 158, rue de**
**l'Université**
**F-75340 Paris Cédex 07 (FR)**

(73) Titulaire : **VIRBAC S.A.**
**1ère Avenue 2 065 m, L.I.D.**
**F-06516 Carros Cedex (FR)**

## Description

La présente invention concerne un nouveau vaccin antirabique.

Le virus rabique est un rhabdovirus constitué de cinq protéines, dont une protéine externe, la glycoprotéine, qui déclenche la synthèse d'anticorps neutralisants chez les animaux inoculés. L'injection de la glycoprotéine purifiée protège l'animal contre une surinfection. Les souches du virus rabique les plus utilisées, notamment la souche CVS et la souche ERA, dont dérivent les souches SAD, telles que les souches SAD Berne et SAD B19 sont décrites dans "Rabies Viruses" par H.F. Clark et T.J. Wiktor - Strains of Human Viruses édité par Majer et Plotkin Karger, Bâle, 1972, p. 177-182 . La séquence en acides aminés de la glycoprotéine de la souche CVS a été décrite par Yelverton et al "Rabies virus glycoprotein analogs : biosynthesis in Escherichia coli" Science 219, 614-620.

Cette glycoprotéine comporte trois sites antigéniques distincts (sites I, II et III), le site III en position 330-340 contient l'arginine 333 qui détermine la virulence de cette souche. D'autres travaux ont par ailleurs montré, dans le cas de la souche CVS, que la lysine pouvait être substituée à l'arginine sans faire perdre le pouvoir pathogène du virus.

La séquence en acides aminés de la glycoprotéine de la souche SAD n'a pas été entièrement établie. Toutefois, on a pu déterminer que le site antigénique III de la glycoprotéine de la souche SAD Berne est identique à celui de la glycoprotéine de la souche CVS.

Les vaccins antirabiques actuellement utilisés sont soit des vaccins fabriqués à partir de virus inactivés, soit des vaccins constitués de souches virales dont la virulence a été atténuée.

L'inactivation des virus peut être réalisée par différents procédés, notamment par des procédés chimiques, tels que le traitement au formol ou à la β-propiolactone.

L'inconvénient majeur de ce procédé de fabrication de vaccins est la manipulation de souches virulentes qui nécessite des conditions de mise en oeuvre très strictes et présente des risques de contamination du personnel de fabrication.

D'autre part, les vaccins inactivés administrés par voie orale n'ont pas de pouvoir protecteur.

L'atténuation de la virulence des souches virales est une technique bien connue ; elle peut être réalisée par exemple par passages successifs des souches virales sur un hôte différent de l'espèce vecteur (lapin ou souris par exemple) ou en culture de cellules. On obtient ainsi des souches désadaptées à l'hôte d'origine et donc moins pathogènes pour celui-ci tout en gardant leur capacité vaccinante.

Les souches SAD, telles que les souches SAD B19 et SAD Berne accessibles au public, sont des souches naturellement atténuées qui ont déjà été testées en Europe pour la vaccination des renards. Elles peuvent être mélangées aux appâts pour une administration par voie orale. Toutefois, ces souches se sont montrées pathogènes pour d'autres espèces animales et l'homme. Il existe donc un risque potentiel de contamination qui diminue considérablement l'intérêt de ces souches pour la vaccination orale.

En effet, par exemple l'administration par voie orale de la souche SAD Berne à un lot de 23 rongeurs sauvages, choisis parmi Apodemus flavicolus et sylvaticus, Arvicola terrestris, Clethrionomys clareolus, Minotus agresti, a provoqué la mort par la rage de deux animaux.

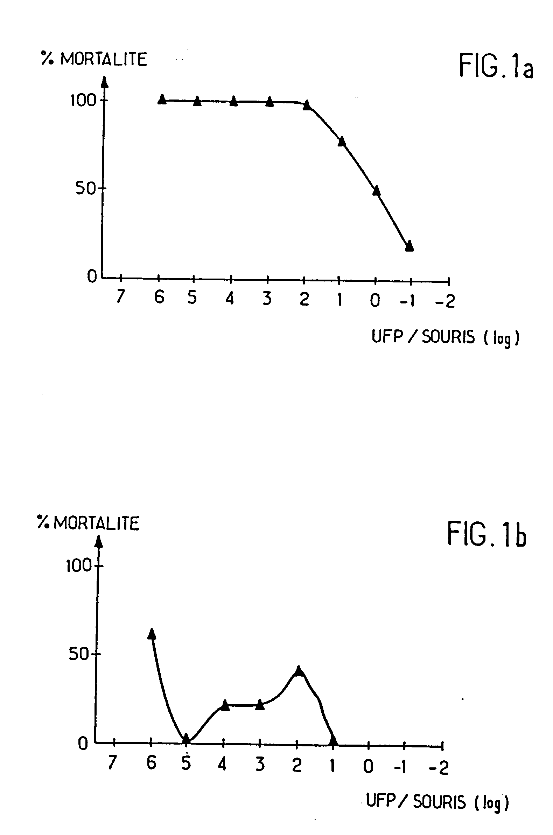

Des essais sur la souris ont également montré que la souche SAD Berne est pathogène pour cette espèce aussi bien par voie intra-cérébrale que par voie intra-musculaire, comme le montrent les courbes des figures 1a et 1b ci-jointes qui sont respectivement:

- Figure 1a : Courbe de mortalité chez les souris adultes en fonction des doses administrées (en unités formant plage) par voie intra-cérébrale.
- Figure 1b : Courbe de mortalité chez les souris adultes en fonction des doses administrées (en unités formant plage) par voie intra-musculaire.

Par voie orale, on a noté chez la souris une mortalité de 10 % à la dose de $10^{5,5}$ UFP.

La souche SAD Berne telle qu'elle ne peut donc pas être utilisée pour une campagne de vaccination des renards sans mettre en danger la faune sauvage et l'homme. Il en est de même de la souche SAD B19.

Il est donc indispensable de trouver un vaccin antirabique qui puisse être utilisé pour l'administration par voie orale tout en étant inoffensif pour les autres espèces animales.

La présente invention concerne un vaccin efficace qui permet de pallier les inconvénients ci-dessus.

Le vaccin selon l'invention est constitué d'un mutant avirulent d'une souche SAD du virus rabique qui est caractérisé par le fait qu'il possède à la place de l'arginine 333 de la glycoprotéine un acide aminé naturel autre que la lysine.

On sait que la substitution de l'arginine 333 par l'isoleucine, la glycine ou la glutamine dans la glycoprotéine de la souche CVS ou de la souche ERA modifie la virulence de cette souche. Toutefois, il n'était pas du tout évident qu'une telle mutation chez une souche SAD, telle que SAD Berne par exemple supprimerait aussi la virulence de celle-ci tout en lui conservant un pouvoir immunogène au moins aussi élevé que la souche mère.

2

Les mutants qui conviennent aux fins de l'invention sont des mutants avirulents et à haut pouvoir protecteur, sélectionnés à partir d'une souche SAD du virus rabique, à l'aide d'anticorps monoclonaux neutralisants.

Les anticorps monoclonaux mis en oeuvre pour sélectionner les mutants selon l'invention sont obtenus par fusion de cellules de myélome et de cellules produisant des anticorps antiviraux selon la technique de l'hybridation décrite par KOHLER et MILSTEIN dans NATURE, vol. 256, 495-497 (1975), technique maintenant bien connue de l'homme de l'art.

Selon cette technique on peut fusionner des cellules provenant d'espèces différentes, toutefois, il est avantageux d'utiliser des cellules provenant de la même espèce animale. Par exemple on utilise de préférence d'unepart des cellules de myélome de souris et d'autre part des cellules de rate de souris préalablement immunisées avec une souche du virus de la rage selon le protocole défini ci-après.

Sous son aspect général, ce procédé d'hybridation décrit en référence aux cellules de souris, comprend les étapes suivantes:

1) d'immunisation de souris avec une quantité donnée de virus inactivé à la ß-propiolactone :

2) de prélèvement de la rate des souris immunisées et séparation des splénocytes ;

3) de fusion des splénocytes ainsi obtenus avec des cellules de myélome de souris en présence d'un promoteur de fusion ;

4) de culture des cellules hybrides obtenues dans un milieu sélectif sur lequel ne se développent pas les cellules de myélomes non fusionnées, et en présence d'éléments nutritifs appropriés ;

5) de sélection des cellules produisant l'anticorps désiré et clonage de ces cellules.

Le protocole d'immunisation comprend l'injection à des souris Balb-C de 100 μg de virus inactivé à la ß-propiolactone par voie intrapéritonéale avec adjuvant complet de FREUND et un rappel par voie intraveineuse 4 jours avant la fusion après une période de repos de 12 mois.

Les splénocytes des souris immunisées sont récupérés après prélèvement de la rate selon le mode opératoire classique.

Les cellules de myélome de souris utilisées pour l'obtention des anticorps monoclonaux neutralisants sont les cellules de myélome de souris Balb-C provenant de la lignée $SP_2O$. Ces cellules de myélome ont été sélectionnées pour leur sensibilité à l'aminoptérine et cultivées sur un milieu approprié, tel que le milieu essentiel d'Eagle modifié par DULBECCO (Dulbecco Modified Eagle medium) dénommé ci-après milieu DMEM, auquel on a ajouté 15 % de sérum de poulain.

La fusion des cellules de myélome avec les splénocytes a été réalisée par mélange de $5.10^5$ cellules de myélome avec $5.10^5$ cellules de rate de souris immunisées, en présence d'un promoteur de fusion, tel que par exemple un polyéthylène glycol.

Après incubation à 37°C, les cellules sont lavées dans le milieu DMEM et remises en suspension, puis cultivées sur un milieu sélectif approprié uniquement pour la croissance des cellules hybrides. Un tel milieu contient de l'aminoxanthine, de l'aminoptérine et de la thymidine.

On sélectionne ensuite les surnageants des cultures, 7 à 20 jours après la fusion, en mettant en contact les surnageants avec une suspension de virus CVS et en retenant les anticorps qui neutralisent ladite suspension.

Parmi ces anticorps on retient ensuite les anticorps qui ne neutralisent pas le mutant avirulent TAG1 dérivant de la souche CVS, déposé à la Collection Nationale de Cultures de Microorganismes (C.N.C.M.) INSTITUT PASTEUR - FRANCE le 12 Avril 1985 sous le n° I-433.

Les anticorps monoclonaux résultants, qui neutralisent donc la souche CVS mais ne neutralisent pas le mutant avirulent TAG1 permettent de sélectionner des mutants avirulents à partir de toute souche de virus de la rage, qui est neutralisée par ces anticorps monoclonaux.

Les mutants selon l'invention sont des mutants avirulents d'une souche SAD, telle que notamment la souche SAD Berne, résistants aux anticorps monoclonaux obtenus selon le procédé ci-dessus.

Le pouvoir pathogène de ces mutants est ensuite testé par injection intracérébrale de $10^5$ UFP à des souris adultes. Les mutants qui ne tuent pas à cette dose et par cette voie d'injection sont considérés comme avirulents.

Ainsi, les mutants selon l'invention sont sélectionnés à partir d'une souche SAD du virus rabique à l'aide des anticorps monoclonaux définis ci-dessus et leur avirulence est contrôlée par le mode opératoire ci-dessus.

Les mutants selon l'invention peuvent en outre contenir d'autres mutations, comme par exemple, la résistance à un anticorps monoclonal neutralisant les souches SAD virulentes, ce qui permet de reconnaître un éventuel revertant de virulence des souches SAD utilisées pour la vaccination orale des renards.

Les mutants selon l'invention peuvent être multipliés sur cellules de rein de hamster nouveaux-nés BHK 21, en présence de milieu GEM (milieu essentiel minimum-modification Glasgow commercialisé par FLOW) et de 2 % de sérum de veau 33° C et en atmosphère humide avec 5 % de $CO_2$.

On les titre selon les méthodes habituelles, par exemple par détermination de la dose létale 50 ($DL_{50}$) chez

3

les souriceaux, par immunofluorescence ou détermination des plages formant lyse sous agar. Ils peuvent être conservés à -70° C.

L'analyse de la séquence en acides aminés de la glycoprotéine de ces mutants a révélé que l'arginine en 333 était remplacée par un acide aminé naturel autre que la lysine. Cet acide aminé peut être par exemple la glutamine, la glycine, l'isoleucine, la leucine, la sérine et la méthionine.

Cette mutation dérive d'un seul changement de nucléotide dans le codon de l'arginine 333. Du fait que les mutations correspondant à des transversions sont dix fois moins fréquentes que les mutations correspondant à des transitions on préfère les mutants dont la mutation résulte d'une transversion. En effet, la fréquence de reversion d'un mutant avirulent dérivant d'une transversion est plus faible que s'il dérive d'une transition.

Le mutant portant une sérine à la place de l'arginine, (mutation correspondant à une transversion) obtenu selon le procédé ci-dessus à partir de la souche SAD Berne et dénommé ci-après SAG1 a été déposé à la Collection Nationale de Cultures de Microorganismes (C.N.C.M.) INSTITUT PASTEUR - FRANCE le 5 juillet 1988 sous le n° I-777. Ce mutant contient une seconde mutation, à savoir la résistance à un anticorps monoclonal neutralisant les souches SAD virulentes obtenu selon le procédé classique de fabrication des hybridomes.

L'invention va être maintenant décrite plus en détail en référence, au mutant SAG1 sans pour autant limiter la portée de celle-ci à ce seul mutant.

A - Tests de mortalité.

1) chez la souris adulte

Le mutant SAG1 a été testé pour son avirulence par injection par voie intra-cérébrale de $10^5$ UFP à des souris adultes, lesquelles ont survécu à cette injection. Ce mutant est donc avirulent.

D'autre part, l'injection orale de SAG1 n'a entraîné aucune mortalité comme le montrent les résultats du tableau ci-après:

| SAG1 par voie orale (UFP) log | mortalité chez la souris adulte |
|---|---|
| 5,5 | 0 |
| 4,5 | 0 |
| 3,5 | 0 |

2) chez les rongeurs Apodemus flavicolus et sylvaticus

Le mutant SAG1 a été administré par voie orale à 20 rongeurs Apodemus flavicolus et sylvaticus à la dose de $10^5$ UFP. Aucune mortalité n'a été provoquée par l'administration de cette dose.

B- efficacité du mutant SAG1 pour la vaccination orale du renard.

Des lots de 4 renards ont été éprouvés par voie intra-musculaire à J 30 avec $10^{3.4}$ UFP de la souche sauvage GS.

Les résultats obtenus figurent dans le tableau ci-après. Ils montrent que le mutant SAG1 a un pouvoir vaccinant semblable à la souche SAD Berne.

| | Dose vaccinales en UFP | Mortalité en % | Titre moyen, à J 30, en anticorps neutralisants |
|---|---|---|---|
| Mutant | $1,8 \times 10^8$ | 0 | 2,65 |
| SAG1 | $1,8 \times 10^7$ | 0 | 2,15 |
| SAD Berne | $4 \times 10^6$ | 0 | 2,34 |
| Témoins | / | 100 | 0 |

Les mutants selon l'invention peuvent être administrés à titre de vaccin vivant par tous les modes d'administration habituellement utilisés pour la vaccination et notamment par voie intra-musculaire ou orale. Les mutants sont avantageusement dilués dans un véhicule inerte pharmaceutiquement acceptable, tel que le sérum physiologique.

**Revendications**

**Revendications pour les Etats contractants suivants : AT, BE, CH, DE, IT, LI, LU, NL, SE**

1. Vaccin antirabique avirulent, caractérisé en ce qu'il est constitué d'un mutant avirulent d'une souche SAD du virus rabique dont la glycoprotéine possède en position 333 un acide aminé naturel autre que la lysine ou l'arginine.

2. Vaccin selon la revendication 1, caractérisé en ce que l'acide aminé en position 333 est choisi parmi la méthionine, la glutamine, la glycine, l'isoleucine, la leucine, ou la sérine.

3. Vaccin selon l'une des revendications 1 ou 2, caractérisé en ce que l'acide aminé en position 333 est la sérine.

4. Vaccin selon l'une quelconque des revendications 1 à 3, caractérisé en ce que le mutant est un mutant de la souche SAD Berne.

5. Vaccin selon l'une quelconque des revendications 1 à 4, caractérisé en ce que le mutant est le mutant déposé à la Collection Nationale de Cultures de Microorganismes - INSTITUT PASTEUR (C.N.C.M.) FRANCE le 5 juillet 1988 sous le n° I-777.

6. Mutant avirulent de la souche SAD Berne déposé à la Collection Nationale de Cultures de Microorganismes (C.N.C.M.) - INSTITUT PASTEUR - FRANCE le 5 juillet 1988 sous le n° I-777.

**Revendications pour les Etats contractants suivants : ES, GR**

1. Procédé pour l'obtention d'un vaccin antirabique avirulent, caractérisé en ce qu'il consiste à utiliser un mutant avirulent d'une souche SAD du virus rabique dont la glycoprotéine possède en position 333 un acide aminé naturel autre que la lysine ou l'arginine.

2. Procédé selon la revendication 1, caractérisé en ce que l'acide aminé en position 333 est choisi parmi la méthionine, la glutamine, la glycine, l'isoleucine, la leucine, ou la sérine.

EP 0 350 398 B1

3. Procédé selon l'une des revendications 1 ou 2, caractérisé en ce que l'acide aminé en position 333 est la sérine.

4. Procédé selon l'une quelconque des revendications 1 à 3, caractérisé en ce que le mutant est un mutant de la souche SAD Berne.

5. Procédé selon l'une quelconque des revendications 1 à 4, caractérisé en ce que le mutant est le mutant déposé à la Collection Nationale de Cultures de Microorganismes -INSTITUT PASTEUR (C.N.C.M.) FRANCE le 5 juillet 1988 sous le n°I-777.

6. Mutant avirulent de la souche SAD Berne déposé à la Collection Nationale de Cultures de Microorganismes (C.N.C.M.) - INSTITUT PASTEUR - FRANCE le 5 juillet 1988 sous le n°I-777.

**Patentansprüche**

**Patentansprüche für folgende Vertragsstaaten : AT, BE, CH, DE, GB, IT, LI, LU, NL, SE**

1. Avirulenter Tollwut-Impfstoff, dadurch gekennzeichnet, daß er aus einem avirulenten mutierten Virus eines desaktivierten, avirulenten Stamms (SAD-Stamm) des Tollwutvirus besteht, dessen Glycoprotein auf Position 333 eine andere natürliche Aminosäure als Lysin oder Arginin besitzt.

2. Impfstoff nach Anspruch 1, dadurch gekennzeichnet, daß die Aminosäure auf Position 333 Methionin, Glutamin, Glycin, Isoleucin, Leucin oder Serin ist.

3. Impfstoff nach einem der Ansprüche 1 oder 2, dadurch gekennzeichnet, daß die Aminosäure auf Position 333 Serin ist.

4. Impfstoff nach einem der Ansprüche 1 bis 3, dadurch gekennzeichnet, daß das mutierte Virus ein Virus des SAD-Stamms Bern ist.

5. Impfstoff nach einem der Ansprüche 1 bis 4, dadurch gekennzeichnet, daß das mutierte Virus das am 5, Juli 1988 unter der Nr. I-777 bei der Collection Nationale de Cultures de Microorganismes - INSTITUT PASTEUR (C.N.C.M) FRANCE eingereichte mutierte Virus ist.

6. Avirulentes mutiertes Virus des SAD-Stamms Bern, unter der Nr. I-777 eingereicht am 5, Juli 1988 bei der Collection Nationale de Cultures de Microorganismes - INSTITUT PASTEUR (C.N.C.M) FRANCE.

**Patentansprüche für folgende Vertragsstaaten : ES, GR**

1. Verfahren für den Erhalt eines avirulenten Tollwut-Impfstoffs, dadurch gekennzeichnet, daß es darin besteht, ein avirulentes mutiertes Virus eines desaktivierten, avirulenten Stamms (SAD-Stamm) des Tollwutvirus zu verwenden, dessen Glycoprotein auf Position 333 eine andere natürliche Aminosäure als Lysin oder Arginin besitzt.

2. Verfahren nach Anspruch 1, dadurch gekennzeichnet, daß die Aminosäure auf Position 333 Methionin, Glutamin, Glycin, Isoleucin, Leucin oder Serin ist.

3. Verfahren nach einem der Ansprüche 1 oder 2, dadurch gekennzeichnet, daß die Aminosäure auf Position 333 Serin ist.

4. Verfahren nach einem der Ansprüche 1 bis 3, dadurch gekennzeichnet, daß das mutierte Virus ein Virus des SAD-Stamms Bern ist.

5. Verfahren nach einem der Ansprüche 1 bis 4, dadurch gekennzeichnet, daß das mutierte Virus das am 5, Juli 1988 unter der Nr. I-777 bei der Collection Nationale de Cultures de Microorganismes - INSTITUT PASTEUR (C.N.C.M) FRANCE eingereichte mutierte Virus ist.

6. Avirulentes mutiertes Virus des SAD-Stamms Bern, unter der Nr. I-777 am 5, Juli 1988 bei der Collection Nationale de Cultures de Microorganismes - INSTITUT PASTEUR (C.N.C.M) FRANCE eingereicht.

## Claims

**Claims for the following Contracting States : AT, BE, CH, DE, GB, IT, LI, LU, NL, SE**

1. Avirulent anti-rabies vaccine, characterized in that it consists of an avirulent mutant of SAD strain of the rabies virus, the glycoprotein of which possesses at position 333 a naturally occurring amino acid other than lysine or arginine.

2. Vaccine according to claim 1, characterized in that the amino acid at position 333 is selected from methionine, glutamine, glycine, isoleucine, leucine or serine.

3. Vaccine according to one of claims 1 or 2, characterized in that the amino acid at position 333 is serine.

4. Vaccine according to any one of claims 1 to 3, characterized in that the mutant is a mutant of the SAD Berne strain.

5. Vaccine according to any one of claims 1 to 4, characterized in that the mutant is the mutant deposited at the National Collection of Cultures of Microorganisms (N.C.C.M.) - PASTEUR INSTITUTE - FRANCE on July 5, 1988 under No. I-777.

6. Avirulent mutant of the SAD Berne strain deposited at the National Collection of Cultures of Microorganisms (N.C.C.M.)-PASTEUR INSTITUTE - FRANCE on July 5, 1988 under No. I-777.

**Claims for the following Contracting States : ES, GR**

1. Method of preparing an avirulent anti-rabies vaccine, characterized in that it consists in using an avirulent mutant of SAD strain of the rabies virus, the glycoprotein of which possesses at position 333 a naturally occurring amino acid other than lysine or arginine.

2. Method according to claim 1, characterized in that the amino acid at position 333 is selected from methionine, glutamine, glycine, isoleucine, leucine or serine.

3. Method according to one of claims 1 or 2, characterized in that the amino acid at position 333 is serine.

4. Method according to any one of claims 1 to 3, characterized in that the mutant is a mutant of the SAD Berne strain.

5. Method according to any one of claims 1 to 4, characterized in that the mutant is the mutant deposited at the National Collection of Cultures of Microorganisms (N.C.C.M.) - PASTEUR INSTITUTE - FRANCE on July 5, 1988 under No. I-777.

6. Avirulent mutant of the SAD Berne strain deposited at the National Collection of Cultures of Microorganisms (N.C.C.M.)-PASTEUR INSTITUTE - FRANCE on July 5, 1988 under No. I-777.

FIG.1a

FIG.1b